# EUROPEAN PATENT SPECIFICATION

(11) **EP 3 894 424 B1**
(45) Date of publication and mention of the grant of the patent: **25.01.2023**
(21) Application number: 19823817.2
(22) Date of filing: 09.12.2019
(51) Int. Cl.: C07J 63/00, A61K 31/58, A61P 31/18

(54) **MESYLATE SALT OF AN AMINO-LUPANE COMPOUND WITH HIV MATURATION INHIBITORY ACTIVITY**
MESYLATSALZ EINER AMINO-LUPAN-VERBINDUNG MIT HIV-REIFUNGSHEMMENDER WIRKUNG
SEL DE MÉSYLATE D'UN COMPOSÉ AMINO-LUPANE AYANT UNE ACTIVITÉ INHIBITRICE DE LA MATURATION DU VIH

(30) Priority: 10.12.2018 US 201862777432 P; 16.07.2019 US 201962874557 P
(43) Date of publication of application: 20.10.2021
(73) Proprietor: VIIV Healthcare UK(No.4) Limited, Brentford, TW8 9GS (GB)
(72) Inventor: WOODS, Amy, Stevenage, Hertfordshire SG1 2NY (GB)
(74) Representative: Matley, Joshua Ellis
(86) International application number: PCT/IB2019/060564
(87) International publication number: WO 2020/121161

(56) References cited:
- WO-A1-2015/157483
- US-B2- 9 527 882
- "Chapters 5-8" In: H. Stahl, C. Wermuth: "Handbook of Pharmaceutical Salts, Ed. 1: Properties Selection and Use", 2002, VHCA, WIley VCH, Zurich, Switzerland ISBN: 3-906390-26-8
- David P. Elder ET AL: "The utility of sulfonate salts in drug development", Journal of Pharmaceutical Sciences, vol. 99, no. 7, 1 January 2010 (2010-01-01), pages 2948-2961, XP055091305, US ISSN: 0022-3549, DOI: 10.1002/jps.22058
- J. M. Miller et al: "Solvent Systems and Their Selection in Pharmaceutics and Biopharmaceutics: Solvent Systems for Crystallization and Polymorph Selection", 2007, Springer, US ISBN: 978-0-387-69154-1 vol. 6, pages 53-109, DOI: 10.1007/978-0-387-69154-1_3,
- GOKHALE M AND MANTRI R ET AL: "Chapter 4: API Solid Form Screening and Selection", DEVELOPING SOLID ORAL DOSAGE FORMS: PHARMACEUTICAL THEORY AND PRACTIE, ACADEMIC PRESS, GB, PAGE(S) 85 - 112 , 1 January 2017 (2017-01-01), XP009528824, ISBN: 978-0-12-802637-3 Retrieved from the Internet: URL:https://api.elsevier.com/content/artic le/PII:B9780128024478000042?httpAccept=tex t/plain

## Description

### FIELD OF THE INVENTION

The present invention relates to compounds, pharmaceutically acceptable salts thereof, pharmaceutical compositions, and methods of use thereof for (i) inhibiting HIV replication in a subject infected with HIV, or (ii) treating a subject infected with HIV.

### BACKGROUND OF THE INVENTION

Human immunodeficiency virus type 1 (HIV-1) infection leads to the contraction of acquired immune deficiency disease (AIDS). The number of cases of HIV infection continues to rise, and currently over twenty-five million individuals worldwide suffer from HIV viral infection. Presently, long-term suppression of viral replication with antiretroviral drugs is the only option for treating HIV-1 infection.

The HIV Gag polyprotein precursor (Pr55Gag), which is composed of four protein domains - matrix (MA), capsid (CA), nucleocapsid (NC) and p6 - and two spacer peptides, SP1 and SP2, represents a new therapeutic target. Although the cleavage of the Gag polyprotein plays a central role in the progression of infectious virus particle production, to date, no antiretroviral drug has been approved for this mechanism.

In most cell types, assembly occurs at the plasma membrane, and the MA domain of Gag mediates membrane binding. Assembly is completed by budding of the immature particle from the cell. Concomitant with particle release, the virally encoded PR cleaves Gag into the four mature protein domains, MA, CA, NC and p6, and the two spacer peptides, SP1 and SP2. Gag-Pol is also cleaved by PR, liberating the viral enzymes PR, RT and IN. Gag proteolytic processing induces a morphological rearrangement within the particle, known as maturation. Maturation converts the immature, donut-shaped particle to the mature virion, which contains a condensed conical core composed of a CA shell surrounding the viral RNA genome in a complex with NC and the viral enzymes RT and IN. Maturation prepares the virus for infection of a new cell and is absolutely essential for particle infectivity.

US Patent 9,527,882 discloses the compound depicted below, which is an inhibitor of HIV maturation and which has utility for the treatment of HIV infection. This compound is : (1*R*)-4-[(1*R*,3a*S*,5a*R*,5b*R*,7a*R*,11a*S*,11b*R*,13a*R*,13b*R*)-3a-{[2-(1,1-dioxido-4-thiomorpholinyl)ethyl]amino}-5a,5b,8,8,11a-pentamethyl-1-(1-propen-2-yl)-2,3,3a,4,5,5a,5b,6,7,7a,8,11,11a,11b,12,13,13a,13b-octadecahydro-1*H-*cyclopenta[*a*]chrysen-9-yl]-1-(fluoromethyl)-3-cyclohexene-1-carboxylic acid and is Example 2b in the `882 patent. This compound may be referred to in the present application as "Compound A".

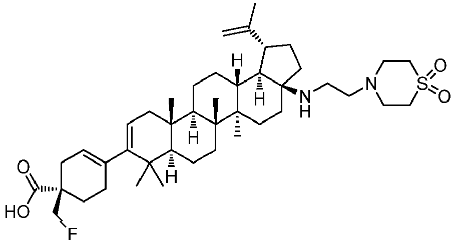

### BRIEF SUMMARY OF THE INVENTION

Briefly, in one aspect, the present invention provides a mesylate salt of Compound A:

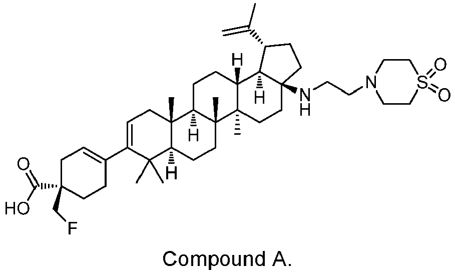

In another aspect, the present invention provides pharmaceutical compositions comprising a mesylate salt of compound A.

In another aspect, the present invention provides pharmaceutical compositions comprising the above salt of compound A for use in treating HIV infection in a human comprising administering a mesylate salt of Compound A to a human patient in need thereof.

In another aspect, the present invention provides a mesylate salt of Compound A for use in therapy.

### BRIEF DESCRIPTION OF THE FIGURES

Figure 1 is an X-Ray Powder Diffraction of an anhydrous crystalline form of the mesylate salt of Compound A.
Figure 2 is a Differential Scanning Colorimetry of an anhydrous crystalline form of the mesylate salt of Compound A.
Figure 3 is a Polarised Light Microscopy image an anhydrous crystalline form of the mesylate salt of Compound A.

### DETAILED DESCRIPTION OF THE INVENTION

Preferably, the mesylate salt of Compound A is crystalline.

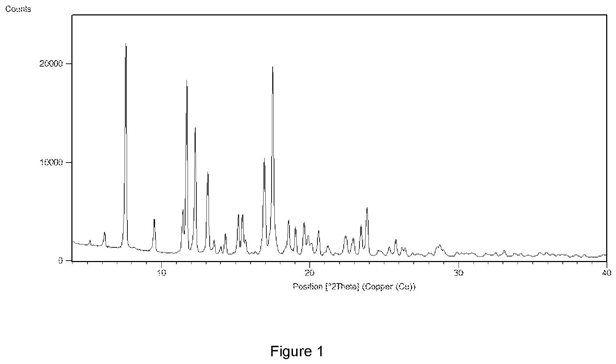

Preferably, the mesylate salt of Compound A is crystalline and anhydrous. In one embodiment, the invention provides an anhydrous crystalline form of a mesylate salt of Compound A, characterized in that it provides an XRPD pattern substantially in accordance with Figure 1. In another embodiment, the invention provides an anhydrous crystalline form of the mesylate salt of Compound A, characterized in that it provides an XRPD pattern substantially as set out in Table 2

**Table 2**

| **20 (°)** | **d-spacings (Å)** |
|---|---|
| **7.6+0.1** | **11.6** |
| **9.5+0.1** | **9.3** |
| **11.4+0.1** | **7.7** |
| **11.7+0.1** | **7.6** |
| **12.3+0.1** | **7.2** |
| **13.1 +0.1** | **6.7** |
| **15.2+0.1** | **5.8** |
| **15.5+0.1** | **5.7** |
| **16.9+0.1** | **5.2** |
| **17.5+0.1** | **5.1** |
| **23.9+0.1** | **3.7** |

In another embodiment, the invention provides an anhydrous crystalline form of the mesylate salt of Compound A that is characterized by an XRPD pattern that has the representative diffraction peaks, in ^{o}2⊖: 7.6±0.3, 9.5±0.3, 11.4±0.3, 11.7±0.3, 12.3±0.3, and 13.1+0.3.

In one embodiment, the pharmaceutical compositions of this invention further comprise pharmaceutically acceptable excipients. In the method of this invention, preferred routes of administration are oral and by injection to deliver subcutaneously. Therefore, preferred pharmaceutical compositions of this invention will be suitable for preparing various dosage forms include tablets, capsules, and forms suitable for injection. Suitable dosage forms will contain between 20 and 300 mg of Compound A (based on weight of the free-base). Particularly preferred tablets and capsules will be those suitable for once daily dosing.

Powders suitable for incorporating into tablets or capsules may be prepared by reducing a salt of the invention into a suitable fine size, for example by micronisation, and mixing with a similarly prepared pharmaceutically acceptable excipient. Pharmaceutically acceptable excipients include, for example, glidants, diluents such as microcrystalline cellulose and mannitol, disintigrants such as sodium starch glycolate, binders such as povidone, lubricants such as magnesium stearate, and other suitable excipients. In one embodiment, the pharmaceutical composition is a tablet. Tablets may optionally be coated with a polymer-based film coating.

The salts of the present invention may be employed alone or in combination with other pharmaceutically active agents useful in the prevention or treatment of HIV infection. The salts of the present invention and any other agent(s) may be administered together or separately and, when administered separately, administration may occur simultaneously or sequentially, in any order. The amounts of the salt of the present invention and the other pharmaceutically active agent(s) and the relative timings of administration will be selected in order to achieve the desired combined therapeutic effect. The administration in combination of a salt of the present invention with other treatment agents may be in combination by administration concomitantly in: (1) a unitary pharmaceutical composition including multiple agents; or (2) separate pharmaceutical compositions each including one of the agents. Alternatively, the combination may be administered separately in a sequential manner wherein one treatment agent is administered first and then the other second or vice versa, and the different agents could be administered on different schedules if appropriate. Such sequential administration may be close in time or remote in time. The amounts of the compound of the invention, or salts thereof and the other pharmaceutically active agent(s) and the relative timings of administration will be selected in order to achieve the desired combined therapeutic effect.

Such other agents include, for example, nucleoside HIV reverse transcriptase inhibitors, non-nucleoside HIV reverse transcriptase inhibitors, HIV protease inhibitors, HIV fusion inhibitors, HIV attachment inhibitors, CCR5 inhibitors, CXCR4 inhibitors, HIV budding or maturation inhibitors, and HIV integrase inhibitors. Preferred other agents include, for example, Dolutegravir, bictegravir, lamivudine, Fostemsavir, Cabotegravir, maraviroc, rilpiverine, Reyataz, Tenofovir, Afenamide, EfDA, Doravirine, and Preziata. Particularly preferred other agents include, for example, Dolutegravir, bictegravir, EfDA, lamivudine, Fostemsavir, and Cabotegravir.

### EXAMPLES

Compound A may be prepared, for example, as described in Example 2b in US Patent 9,527,882.

X-Ray Powder Diffraction (XRPD) data were acquired on a Bruker D8 Advance X-ray powder diffractometer. The acquisition conditions were: radiation: Cu Kα, generator voltage: 40 kV, generator current: 40 mA, start angle: 2.0° 2θ, end angle: 40.0° 2θ, step size: 0.02° 2θ, time per step: 0.325 seconds. Samples were prepared by mounting the sample onto a silicon wafer (zero background) plate, resulting in a thin layer of powder.

Differential Scanning Calorimetry (DSC) data were acquired on a TA Instruments Q20 DSC. The method parameters were: Heat Program: 30°C to 300°C using a 10°C per minute heating rate, under a nitrogen gas flow.

Polarised Light Microscopy (PLM) image was acquired using polarised light with a Zeiss Axioplan 2 Microscope.

### First Seed Preparation of Anhydrous Crystalline Mesylate salt of Compound A

Crude Compound A was suspended in 50 volume equivalents ("vol_{eq}") of Ethyl Acetate. The suspension was then heated to 40°C to form a solution. 1 mole equivalent of 3 Molar aqueous methanesulfonic acid was added to the solution at room temperature. The resultant solution was stirred whilst cycling the temperature between 40°C and 5°C for three days. The suspension was then filtered and air dried for 1 hour to afford an anhydrous crystalline form of the mesylate salt of Compound A. The unique and novel crystalline form of the mesylate salt of Compound A was confirmed by X-Ray powder Diffraction ("XRPD") and Differential Scanning Colorimetry ("DSC"). This form is designated as Form 1. This Form 1 of the mesylate salt of Compound A can be used as seeds in future preparations.

### Preparation of Anhydrous Crystalline Mesylate salt of Compound A

Approx. 20 mg of crude Compound A was suspended in approx. 0.9 g of Ethyl Acetate (EtOAc). The suspension was then heated to 40-50 °C to form a solution. Approx. 9 ul of 3 Molar aqueous Methanesulfonic acid (MSA) was added to the solution at room temperature. The resultant solution was stirred whilst cycling the temperature between 40 °C and 5 °C for three days. The suspension was then filtered and air dried for 1 hour to afford anhdrous crystalline Form 1 of the mesylate salt of Compound A. This Form 1 of the mesylate salt of Compound A can be used as seeds in future preparations. The unique and novel crystalline form of the mesylate salt of Compound A was confirmed by X-Ray powder Diffraction ("XRPD") and Differential Scanning Colorimetry ("DSC").

### Preparation of Crystalline Form 1 of a mesylate salt of Compound A

1 g of crude Compound A was suspended in 18.8 g of Ethyl Acetate:Dimethylsulfoxide (39:1, wt:wt). The suspension was then heated to 48-53 °C and held at that temperature until full dissolution was achieved. A clarifying filtration was performed into another vessel and residual solution from transfer devices was rinsed with 0.8 g of Ethyl Acetate:Dimethylsulfoxide (39:1, wt:wt). Next, 0.22 g of Methanesulfonic acid (MSA)/EtOAc solution (MSA/EtOAc=0.18:4.5, wt:wt) was charged to the Compound A solution. The resultant solution was seeded with 0.006 g daughter seeds of the mesylate salt of Compound A Form 1 in 0.3g Ethyl Acetate. The suspension was aged for 1 hour (h) and then 3.7 g of Methanesulfonic acid (MSA)/EtOAc solution (0.18:4.5, wt:wt) was charged over a period of approx. 5 h. The suspension was cooled to 3-8 °C over 5 h then aged for 8 h. The suspension was then filtered under vacuum and the cake was subsequently washed three times with approx. 4 g of Ethyl Acetate. The cake was then dried in vacuum at 20-30 °C for 6 h and then further dried at 48-52 °C for 30 h to afford Form 1 of the mesylate salt of Compound A. The unique and novel crystalline form of the mesylate salt of Compound A was confirmed by X-Ray powder Diffraction ("XRPD") and Differential Scanning Colorimetry ("DSC").

### Preparation of Crystalline Form 1 of a mesylate salt of Compound A

Crude Compound A was suspended in 21 vol_{eq} of 98:2 v/v Ethyl Acetate:Dimethylsulfoxide ("DMSO"). The suspension was then heated to 50°C and held at that temperature until full dissolution was achieved. A clarifying filtration was performed into another vessel and residual solution transferred with 1 vol_{eq} of Ethyl Acetate. The temperature of the solution was maintained at 50°C then 10% weight equivalents ("wt_{eq}") of an acid solution containing 1.1 mole equivalents of methanesulfonic acid in 5 vol_{eq} of Ethyl Acetate was charged to the Compound A solution. The resultant solution was seeded with daughter seeds of 0.5% wt_{eq} of the mesylate salt of Compound A Form 1 in 0.5 vol_{eq} Ethyl Acetate. The suspension was aged for 1 hour ("h") and then the remaining acid solution was charged over a period of 6 h. The suspension was cooled to 5°C over 6 h then aged for 8 h. The suspension was then filtered under vacuum and the cake was subsequently washed with 3x5 vol_{eq} of Ethyl Acetate. The cake was then dried in vacuum at 50°C for 20 h to afford Form 1 of the mesylate salt of Compound A.
Equivalents are based on assay of crude freebase.

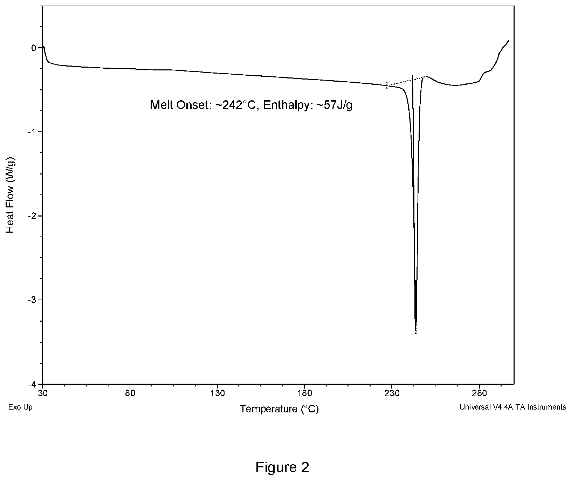

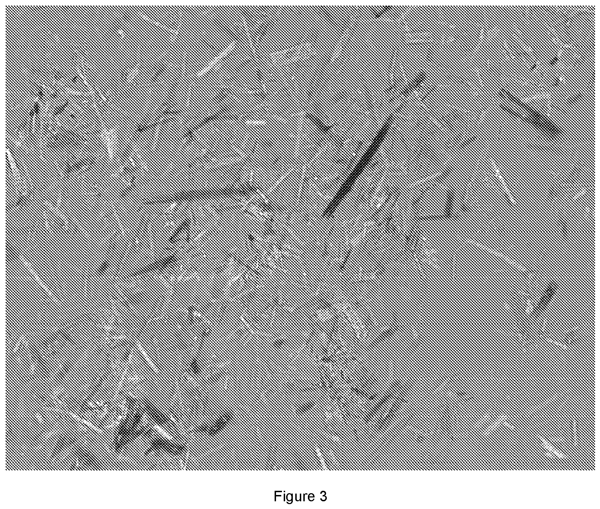

The weight percent of free base and methane sulfonic acid were determined by Ion Chromatography and confirmed a 1/1 molar ratio between methanesulfonic acid and Compound A. The unique and novel crystalline form of the mesylate salt of Compound A was confirmed by X-Ray powder Diffraction ("XRPD") and Differential Scanning Colorimetry ("DSC"). See Figures 1 and 2. Figure 2 shows that Form 1 crystalline mesylate salt of Compound A has an onset of melting at approximately 242°C. Polarized Light Microscopy indicated birefringence confirming the crystallinity of solids of Form 1 mesylate salt of Compound A. See Figure 3.

As with all pharmaceutical compounds and compositions, chemical and physical properties of salts of Compound A are important in its commercial development as a novel HIV therapeutic. These properties include, but are not limited to: (1) packing properties such as molar volume, density and hygroscopicity, (2) thermodynamic properties such as melting temperature, vapor pressure and solubility, (3) kinetic properties such as dissolution rate and stability (including stability at ambient conditions, especially to moisture, and under storage conditions), (4) surface properties such as surface area, wettability, interfacial tension and shape, (5) mechanical properties such as hardness, tensile strength, compactibility, handling, flow and blend; and (6) filtration properties.

We have found that the mesylate salt of Compound A, in particular crystalline Form 1, has certain advantages over the parent free base and over the Bis-HCl salt of Compound A. The Bis-HCl salt of Compound A is disclosed int the `882 patent. The parent free base of Compound A was eliminated as a suitable version for development based on its poor solubility in Fasted State Simulated Intenstinal Fluid ("FaSSIF") and Fed State Simulated Intestinal Fluid ("FeSSIF"). Compared to the bis-HCl salt of Compound A, Form 1 of the mesylate salt of Compound A has greater solubility in both FaSSIF and FeSSIF. The solubility of the parent Compound A, the bis-HCl salt of Compound A and Form 1 of the Mesylate salt of Compound A in various media at controlled ambient room temperature is summarized below in Table 1.

**Table 1**

| | **Solubility of Specified Version (mq/mL)** | | |
|---|---|---|---|
| **Media** | **Parent Compound A** | **Bis-HCl salt of Compound A** | **Form 1 of Mesylate salt of Compound A** |
| Fasted State Simulated Intestinal Fluid (FaSSif) pH 6.5 | Less than 0.001 | Approximately 0.04 | 0.1 |
| Fed State Simulated Intestinal Fluid (FeSSif) pH 6.5 | Less than 0.001 | 0.15 | 0.6 |

In addition, compared to the bis-HCl salt of Compound A, Form 1 of the mesylate salt of Compound A has lower hygroscopicity with no significant form change upon moisture sorption/adsorption in ambient humidity conditions which results in greater physical stability. Water vapor sorption experiments were performed using a Surface Measurement System DVS Advantage at 25°C. The Bis-HCl salt of Compound A reversibly absorbed approximately 7% weight per weight of water between 0% and 90% relative humidity at 25°C. Upon exposure at 40% relative humidity, the Bis-HCl salt of Compound A reversibly absorbed approximately 4% weight per weight of water resulting in a polymorphic form conversion. Upon exposure at 70% relative humidity, the Bis-HCl salt of Compound A reversibly absorbed approximately an additional 3% weight per weight of water resulting in conversion to a transient, hydrated solid state form. Upon desorption the conversions were reversible. The Form 1 crystalline mesylate salt of Compound A reversibly absorbed approximately 1.6% weight per weight of water between 0% and 90% relative humidity at 25°C.

Compared to the bis-HCl salt of Compound A, crystalline Form 1 of the mesylate salt of Compound A has a notably higher melting temperature. Additionally, the bis-HCL salt of Compound A exhibits thermal loss of counter-ion during heating, whereas the mesylate salt of Compound A does not show this behavior. This implies that the Form 1 mesylate salt is thermally and physically more stable than the bis-HCL salt.

### Tablets:

Tablets were prepared using the Form 1 anhydrous crystalline mesylate salt of this invention, microcrystalline cellulose, mannitol, sodium starch glycolate, povidone, and magnesium stearate. The tablets were coated with a polymer-based film coating, Opadry Film Coat, white/OY-S-28876, commercially available from Colorcon. The tablets, which contain 25 mg or 100 mg of the Form 1 anhydrous crystalline mesylate salt of compound A, based on weight of free base, were prepared using granules of the following formulation. Formula quantities are shown as a weight %.
Form 1 rystalline mesylate salt of compound A, active substance - 54.83%
Microcrystalline cellulose, Avicel PH101, diluent - 18.00%
Mannitol, Pearlitol 25C, diluent - 15.17%
Sodium starch glycolate, Glycolys, disintegrant - 7.00%
Povidone, Plasdone K29/32, binder - 5%

Tablets containing 25 mg of the Form 1 mesylate salt of compound A, based on weight of free base, were prepared using the above granules, in the formula below:
Granules - 51.625 mg
Microcrystalline cellulose, Avicel PH102, diluent - 105 mg
Mannitol, Pearlitol 200SD, diluent - 189.88 mg
Magnesium Stearate, Ligamed MF-2-V or MF-2-K Oral, lubricant - 3.50 mg

The resulting tablets were then coated with 10.50 mg of Opadry Film Coat, white OY-S-28876.

Tablets containing 100 mg of the Form 1 mesylate salt of compound A, based on weight of free base, were prepared using the above granules, in the formula below:
Granules - 206.50 mg mg
Microcrystalline cellulose, Avicel PH102, diluent - 105 mg
Mannitol, Pearlitol 200SD, diluent - 35.00 mg
Magnesium Stearate, Ligamed MF-2-V or MF-2-K Oral, lubricant - 3.50 mg

The resulting tablets were then coated with 10.50 mg of Opadry Film Coat, white OY-S-28876.

## Claims

1. A mesylate salt of the compound

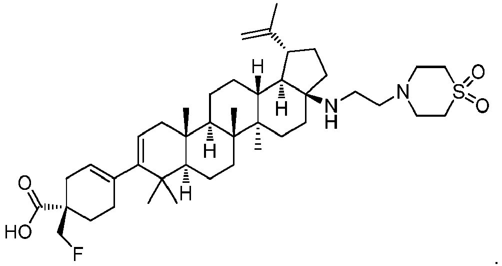

2. A crystalline form of the mesylate salt of Claim 1.

3. An anhydrous crystalline form of the mesylate salt of Claim 1.

4. An anhydrous crystalline form of the mesylate salt of Claim 1, **characterized in that** it provides an XRPD pattern substantially in accordance with Figure 1.

5. An anhydrous crystalline form of the mesylate salt of Claim 1, **characterized in that** it provides an XRPD pattern substantially as set out in Table 2
**Table 2**
| **2θ (°)** | **d-spacings (Å)** |
|---|---|
| **7.6±0.1** | **11.6** |
| **9.5±0.1** | **9.3** |
| **11.4±0.1** | **7.7** |
| **11.7±0.1** | **7.6** |
| **12.3±0.1** | **7.2** |
| **13.1±0.1** | **6.7** |
| **15.2±0.1** | **5.8** |
| **15.5±0.1** | **5.7** |
| **16.9±0.1** | **5.2** |
| **17.5±0.1** | **5.1** |
| **23.9±0.1** | **3.7** |

6. An anhydrous crystalline form of the mesylate salt of Claim 1 that is **characterized by** an XRPD pattern that has the representative diffraction peaks, in ^{o}2⊖: 7.6±0.3, 9.5±0.3, 11.4±0.3, 11.7±0.3, 12.3±0.3, and 13.1±0.3.

7. A pharmaceutical composition comprising a salt according to any of Claims 1-6.

8. A pharmaceutical composition according the Claim 7 further comprising a pharmaceutically acceptable excipient.

9. A pharmaceutical composition according to Claim 8 wherein said composition is in the form of a tablet, a capsule, or a form suitable for injection.

10. A tablet according to Claim 9, further comprising at least one pharmaceutically acceptable excipient selected from the group consisting of microcrystalline cellulose, mannitol, sodium starch glycolate, povidone, and magnesium stearate.

11. A tablet according to Claim 10 wherein said tablet is coated with a polymer-based film coating.

12. A pharmaceutical composition according to any of Claims 7-11 for use in treating an HIV infection in a human.

13. The pharmaceutical composition for use according to Claim 12, wherein the use further comprises administering to the human at least one other agent used for treatment of HIV infection selected from the group consisting of nucleoside HIV reverse transcriptase inhibitors, non-nucleoside HIV reverse transcriptase inhibitors, HIV protease inhibitors, HIV fusion inhibitors, HIV attachment inhibitors, CCR5 inhibitors, CXCR4 inhibitors, HIV budding or maturation inhibitors, and HIV integrase inhibitors.

14. The pharmaceutical composition for use according to Claim 12 wherein said other agent is selected from the group consisting of dolutegravir, bictegravir, and cabotegravir.

15. A salt as defined in any of Claims 1-6 for use in therapy.

## Patentansprüche

1. Mesylatsalz der Verbindung

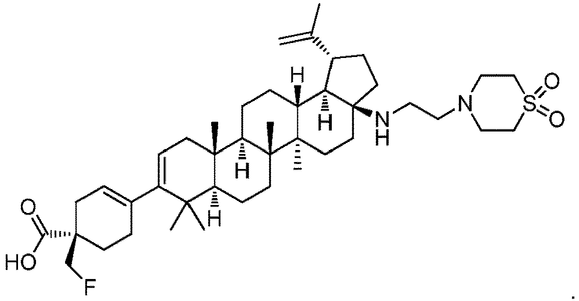

2. Kristalline Form des Mesylatsalzes gemäß Anspruch 1.

3. Wasserfreie kristalline Form des Mesylatsalzes gemäß Anspruch 1.

4. Wasserfreie kristalline Form des Mesylatsalzes gemäß Anspruch 1, **dadurch gekennzeichnet, dass** es ein XRPD-Diagramm ergibt, das im Wesentlichen in Übereinstimmung mit Figur 1 ist.

5. Wasserfreie kristalline Form des Mesylatsalzes gemäß Anspruch 1, **dadurch gekennzeichnet, dass** es ein XRPD-Diagramm ergibt, das im Wesentlichen so wie in Tabelle 2 aufgeführt ist
**Tabelle 2**
| **28 (°)** | **d-Abstände (Ä)** |
|---|---|
| 7,6+0,1 | 11,6 |
| 9,5+0,1 | 9,3 |
| 11,4+0,1 | 7,7 |
| 11,7+0,1 | 7,6 |
| 12,3+0,1 | 7,2 |
| 13,1+0,1 | 6,7 |
| 15,2+0,1 | 5,8 |
| 15,5+0,1 | 5,7 |
| 16, 9+0, 1 | 5,2 |
| 17,5+0,1 | 5,1 |
| 23,9+0,1 | 3,7 |

6. Wasserfreie kristalline Form des Mesylatsalzes gemäß Anspruch 1, das durch ein XRPD-Diagramm gekennzeichnet ist, das die repräsentativen Beugungspeaks in °2θ aufweist: 7,6±0,3, 9,5±0,3, 11,4±0,3, 11,7±0,3, 12,3±0,3 und 13,1±0,3.

7. Pharmazeutische Zusammensetzung, umfassend ein Salz gemäß einem der Ansprüche 1-6.

8. Pharmazeutische Zusammensetzung gemäß Anspruch 7, ferner umfassend einen pharmazeutisch annehmbaren Hilfsstoff.

9. Pharmazeutische Zusammensetzung gemäß Anspruch 8, wobei die Zusammensetzung in Form einer Tablette, einer Kapsel oder einer zur Injektion geeigneten Form vorliegt.

10. Tablette gemäß Anspruch 9, ferner umfassend mindestens einen pharmazeutisch annehmbaren Hilfsstoff, ausgewählt aus der Gruppe, bestehend aus mikrokristalliner Cellulose, Mannit, Natriumstärkeglycolat, Povidon und Magnesiumstearat.

11. Tablette gemäß Anspruch 10, wobei die Tablette mit einer polymerbasierten Filmbeschichtung beschichtet ist.

12. Pharmazeutische Zusammensetzung gemäß einem der Ansprüche 7-11 zur Verwendung in der Behandlung einer HIV-Infektion bei einem Menschen.

13. Pharmazeutische Zusammensetzung zur Verwendung gemäß Anspruch 12, wobei die Verwendung ferner die Verabreichung an einen Menschen von mindestens einem anderen Mittel, das zur Behandlung einer HIV-Infektion verwendet wird, ausgewählt aus der Gruppe, bestehend aus nukleosidischen HIV-Reverse-Transkriptase-Inhibitoren, nicht-nukleosidischen HIF-Reverse-Transkriptase-Inhibitoren, HIV-Proteaseinhibitoren, HIV-Fusionsinhibitoren, HIV-Attachment-Inhibitoren, CCR5-Inhibitoren, CXCR4-Inhibitoren, HIV-Knospungs-oder -Reifungsinhibitoren und HIV-Integrase-Inhibitoren, umfasst.

14. Pharmazeutische Zusammensetzung zur Verwendung gemäß Anspruch 12, wobei das andere Mittel aus der Gruppe, bestehend aus Dolutegravir, Bictegravir und Cabotegravir, ausgewählt ist.

15. Salz wie gemäß einem der Ansprüche 1-6 definiert zur Verwendung in der Therapie.

## Revendications

1. Sel mésylate du composé

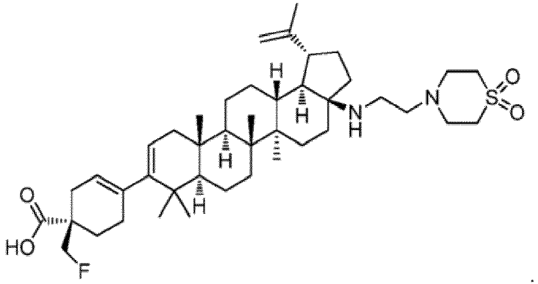

2. Forme cristalline du sel mésylate selon la revendication 1.

3. Forme cristalline anhydre du sel mésylate selon la revendication 1.

4. Forme cristalline anhydre du sel mésylate selon la revendication 1, **caractérisée en ce qu'**elle fournit un diagramme DRXP sensiblement conforme à la figure 1.

5. Forme cristalline anhydre du sel mésylate selon la revendication 1, **caractérisée en ce qu'**elle fournit un diagramme DRXP sensiblement tel qu'indiqué dans le tableau 2.
**Tableau 2**
| 2θ (°) | Distances d (Å) |
|---|---|
| 7,6 ± 0,1 | 11,6 |
| 9,5 ± 0,1 | 9,3 |
| 11,4 ± 0,1 | 7,7 |
| 11,7 ± 0,1 | 7,6 |
| 12,3 ± 0,1 | 7,2 |
| 13,1 ± 0,1 | 6,7 |
| 15,2 ± 0,1 | 5,8 |
| 15,5 ± 0,1 | 5,7 |
| 16,9 ± 0,1 | 5,2 |
| 17,5 ± 0,1 | 5,1 |
| 23,9 ± 0,1 | 3,7 |

6. Forme cristalline anhydre du sel mésylate selon la revendication 1 qui est **caractérisée par** un diagramme DRXP qui a les pics de diffraction représentatifs, en °2θ : 7,6 ± 0,3, 9,5 ± 0,3, 11,4 ± 0,3, 11,7 ± 0,3, 12,3 ± 0,3 et 13,1 ± 0,3.

7. Composition pharmaceutique comprenant un sel selon l'une quelconque des revendications 1 à 6.

8. Composition pharmaceutique selon la revendication 7 comprenant en outre un excipient pharmaceutiquement acceptable.

9. Composition pharmaceutique selon la revendication 8, où ladite composition est sous la forme d'un comprimé, d'une gélule ou d'une forme appropriée à l'injection.

10. Comprimé selon la revendication 9, comprenant en outre au moins un excipient pharmaceutiquement acceptable choisi dans le groupe consistant en la cellulose microcristalline, le mannitol, le glycolate d'amidon sodique, la povidone et le stéarate de magnésium.

11. Comprimé selon la revendication 10, où ledit comprimé est enrobé d'un enrobage par film à base de polymère.

12. Composition pharmaceutique selon l'une quelconque des revendications 7 à 11 destinée à être utilisée dans le traitement d'une infection à VIH chez un humain.

13. Composition pharmaceutique destinée à être utilisée selon la revendication 12, où l'utilisation comprend en outre l'administration à l'humain d'au moins un autre agent utilisé pour le traitement d'une infection à VIH choisi dans le groupe consistant en les inhibiteurs de transcriptase inverse de VIH nucléosidiques, les inhibiteurs de transcriptase inverse de VIH non nucléosidiques, les inhibiteurs de protéase de VIH, les inhibiteurs de fusion de VIH, les inhibiteurs de fixation de VIH, les inhibiteurs de CCR5, les inhibiteurs de CXCR4, les inhibiteurs de bourgeonnement ou de maturation de VIH et les inhibiteurs d'intégrase de VIH.

14. Composition pharmaceutique destinée à être utilisée selon la revendication 12, où ledit autre agent est choisi dans le groupe consistant en le dolutégravir, le bictégravir et le cabotégravir.

15. Sel tel que défini dans l'une quelconque des revendications 1 à 6 destiné à être utilisé en thérapie.
